# FASCICULE DE BREVET EUROPEEN

(11) **EP 1 983 932 B1**
(45) Date de publication et mention de la délivrance du brevet: **05.01.2011**
(21) Numéro de dépôt: 07726326.7
(22) Date de dépôt: 08.02.2007
(51) Int. Cl.: A61F 2/84, A61F 2/90

(54) **SYSTEME DE TRAITEMENT DE LESIONS SUR UNE BIFURCATION DE VAISSEAU SANGUIN**
SYSTEM ZUR BEHANDLUNG VON LÄSIONEN AN EINER BLUTGEFÄSSGABELUNG
SYSTEM FOR TREATING LESIONS ON A BLOOD VESSEL BIFURCATION

(30) Priorité: 09.02.2006 FR 0601183
(43) Date de publication de la demande: 29.10.2008
(73) Titulaire: Dibie, Alain, 75015 Paris (FR)
(72) Inventeur: Dibie, Alain, 75015 Paris (FR)
(74) Mandataire: Texier, Christian
(86) Numéro de dépôt international: PCT/EP2007/051205
(87) Numéro de publication internationale: WO 2007/090863

(56) Documents cités:
- WO-A2-02/091951
- WO-A2-2005/096995
- US-A1- 2002 193 873
- US-A1- 2004 088 007

## Description

La présente invention concerne le domaine des systèmes de traitement de lésions sur des bifurcations de vaisseaux sanguins.

La présente invention concerne plus précisément un système de pose d'endoprothèses à cet effet.

On connaît conventionnellement le traitement des sténoses décelées sur des artères coronaires par la mise en place d'endoprothèses formées de corps tubulaire ajourés en grillage et expansibles, grâce à un guide, sur le site sténosé.

Le plus souvent l'expansion de ces endoprothèses est provoquée par gonflage d'un ballon situé à l'intérieur des endoprothèses, le ballon étant retiré par la suite.

Ce système de pose usuel d'une endoprothèse a déjà rendu de grands services. Cependant, il ne donne pas totalement satisfaction.

En particulier, le déposant a constaté qu'il ne donne pas totalement satisfaction lorsque, comme cela est fréquent, la sténose est située au niveau d'une bifurcation de vaisseau sanguin.

En effet, dans ce cas, la disposition de deux endoprothèses distinctes destinées à être placées respectivement dans les différentes branches de la bifurcation et dont le positionnement relatif doit être ajusté au plus près pour couvrir au moins la zone de bifurcation nécessite l'utilisation de deux systèmes de pose distincts au cours de deux procédures réalisées en parallèle.

Ces deux procédures distinctes voient se multiplier les risques de croisement des guides mettant en place les endoprothèses sur la zone de bifurcation, rendant alors complexe le traitement des sites sténosés sur les bifurcations de vaisseaux sanguins.

Par ailleurs, le document US 2002/0193873 propose un système pour délivrer une endoprothèse au niveau sur des bifurcations de vaisseaux sanguins ayant un mécanisme d'engagement unique pour joindre deux corps d'endoprothèses lorsque celles-ci sont dilatées.

Enfin, le document WO 02/091951 décrit un système de traitement des lésions sur des bifurcations de vaisseaux sanguins, dans lequel une endoprothèse présentant une structure géométrique particulière est placée dans les vaisseaux de sorte qu'une fois dilatée, sa portion centrale couvre au moins une partie de l'ouverture d'une branche secondaire. Pour cela, le système de délivrance comporte deux instruments de pose attachés à un arbre commun qui vient en butée au niveau de la bifurcation des branches.

Un but premier de la présente invention est de perfectionner les systèmes de pose des endoprothèses pour faciliter et améliorer le traitement des sténoses sur les bifurcations de vaisseaux sanguins.

Un second but de la présente invention est de proposer un système de pose d' endoprothèses laissant le choix à l'opérateur de poser soit une soit deux endoprothèses dans la zone de bifurcation d'un vaisseau sanguin au cours de la même procédure.

Un autre but de l'invention est de proposer un système de pose limitant les risques de croisement de guides entre deux instruments de pose associés à deux endoprothèses destinées chacune à une branche d'une bifurcation de vaisseau.

Il est également désirable de proposer un système de pose d'endoprothèses simple d'utilisation et de fabrication tout en conservant une efficacité maximale.

Ces buts sont atteints dans le cadre de la présente invention grâce à un système de traitement de lésions sur une bifurcation de vaisseau sanguin selon la revendication 1.

L'invention sera mieux comprise et d'autres avantages et caractéristiques apparaîtront à la lecture de la description qui va suivre donnée à titre d'exemple non limitatif et grâce aux dessins annexés parmi lesquels :
- La figure 1 illustre une vue schématique latérale d'un instrument de pose associé à une endoprothèse principale dédiée à une première branche d'une bifurcation d'un vaisseau sanguin;
- La figure 2 illustre une vue schématique latérale d'un instrument de pose à double ballon associé à une endoprothèse secondaire dédiée à une seconde branche d'une bifurcation d'un vaisseau sanguin;
- Les figures 3a et 3b illustrent des vues en coupe, respectivement, d'une endoprothèse principale dédiée à une première branche d'une bifurcation d'un vaisseau sanguin et d'une endoprothèse secondaire biseautée dédiée à une seconde branche de la bifurcation;
- La figure 4 illustre une vue en perspective des endoprothèses des figures 3a et 3b disposées au niveau de la bifurcation d'un vaisseau sanguin;
- La figure 5a illustre une vue latérale d'un système de pose d'endoprothèses selon l'invention comprenant l'instrument de pose à double ballon de la figure 2 coopérant avec l'instrument de pose de la figure 1 dans une endoprothèse;
- La figure 5b illustre une vue latérale de la mise en place d'un système de pose d'endoprothèses selon l'invention comprenant l'instrument de pose à double ballon de la figure 2 coopérant avec l'instrument de pose de la figure 1;
- La figure 5c illustre une vue latérale du déploiement de l'endoprothèse portée par l'instrument de pose de la figure 1, par inflation d'un premier ballon de l'instrument de pose à double ballon de la figure 2;
- La figure 5d illustre une vue latérale du déploiement de l'endoprothèse portée par l'instrument de pose de la figure 1, par déploiement du ballon de ce dernier ;
- La figure 5e illustre une vue latérale de la mise en place de l'endoprothèse secondaire par l'instrument de pose à double ballon associé dans une branche de la bifurcation d'un vaisseau sanguin;
- La figure 5f illustre une variante de la figure 5c avec l'endoprothèse secondaire déployée;
- Les figures 6a, 6b, 6c illustrent les mouvements relatifs coordonnés des deux instruments de pose d'un système de pose selon l'invention lors de la mise en place d'endoprothèses au niveau de la bifurcation d'un vaisseau sanguin.

### 1. Système de pose d'endoprothèses pour le traitement de lésions sur des bifurcations de vaisseaux

On va tout d'abord décrire un système de pose d'endoprothèses pour le traitement de lésions sur des bifurcations de vaisseaux sanguins conforme à la présente invention.

### a. Les instruments du système de pose

Ce système de pose va permettre de poser soit une soit deux endoprothèses, selon les besoins du vaisseau V à traiter, au cours d'une même procédure.

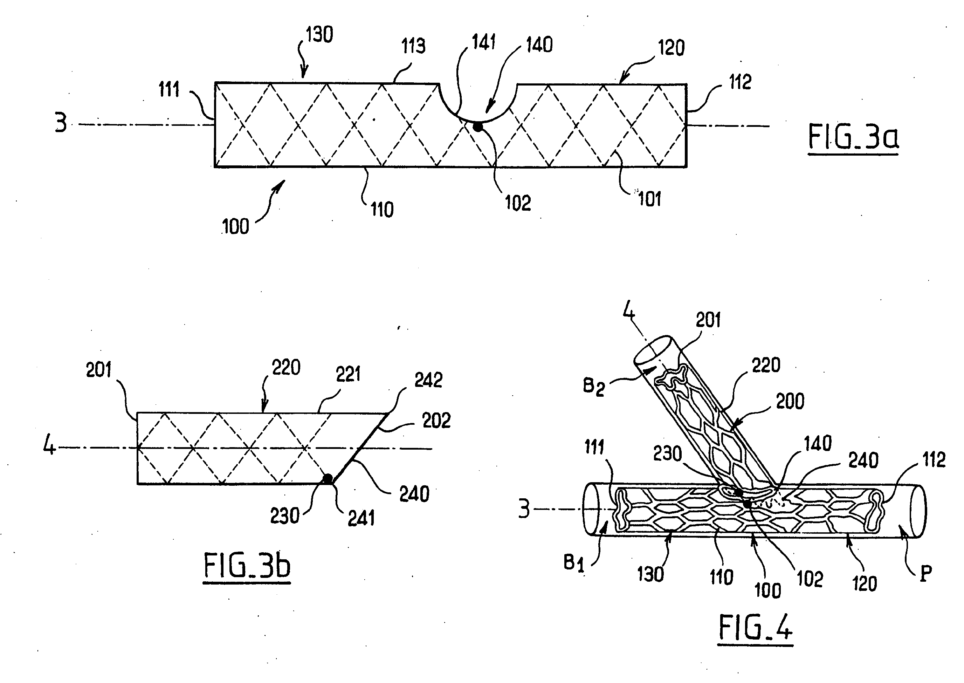

Il comprend, d'une part, un premier instrument de pose 300 présentant un ballon principal 310 prévu pour dilater une endoprothèse dite principale 100 destinée à être placée dans une première branche B1 de la bifurcation et un tronçon principal P, en amont de la bifurcation d'un vaisseau sanguin V à traiter, comme on le voit en particulier sur la figure 4.

Ce ballon principal 310 est associé à une lumière de guidage 323 pouvant recevoir un premier élément de guidage 800 destiné à diriger le ballon dans la première branche B1 de la bifurcation.

D'autre part, le système de pose comprend un second instrument de pose 400 à double ballon dédié à la mise en place éventuelle d'une endoprothèse dite secondaire 200 dans la seconde branche B2 de la bifurcation du vaisseau sanguin V à traiter.

Ce second instrument de pose 400 est prévu pour coopérer avec le premier.

Il comprend un premier ballon 500 adapté pour être introduit dans la deuxième branche B2 à travers un orifice 140 de l'endoprothèse principale 100, le premier ballon 500 étant également adapté pour dilater l'orifice 140 de l'endoprothèse principale 100.

Il comprend également un second ballon 600 adapté pour recevoir l'endoprothèse secondaire 200 et la dilater, ladite endoprothèse étant destinée à être introduite dans la seconde branche (B2) par engagement à travers l'orifice (140) de la première endoprothèse (100), une fois celui-ci dilaté.

D'autre part, il comporte, avantageusement, des moyens de réception 530 pouvant recevoir en partie le premier élément de guidage 800 du premier instrument de pose 300 de façon à maintenir solidaires les deux instruments de pose 300 et 400 pendant la progression du système de pose vers la bifurcation du vaisseau V à traiter.

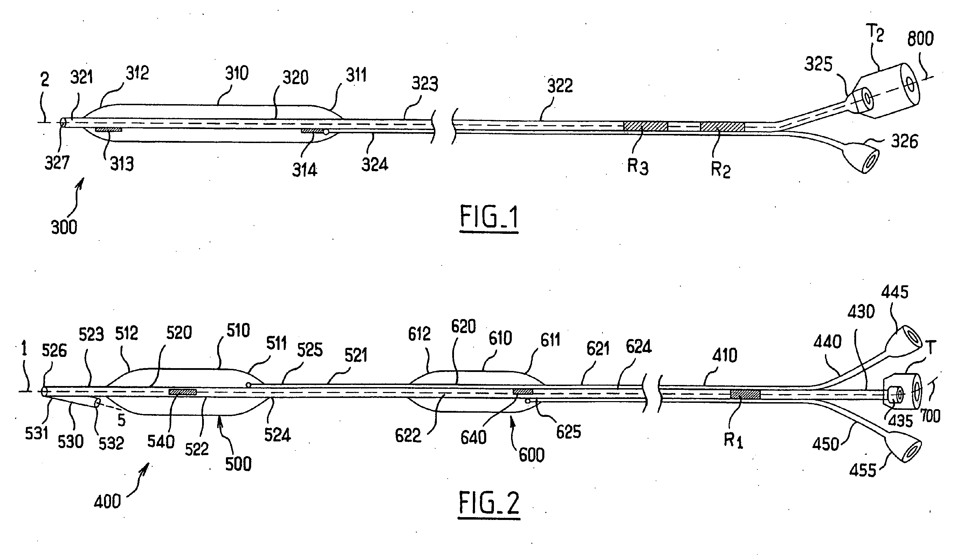

On va maintenant décrire, plus précisément, la structure du second instrument de pose 400 à double ballon, représenté sur la figure 2.

Pour l'essentiel, cet instrument de pose 400, présentant un axe longitudinal 1, comprend le premier 500 et le second ballons 600, le plus proximal, disposés en enfilade le long de cet axe 1.

Ces deux ballons 500 et 600 sont formés, respectivement, de préférence, d'éléments tubulaires 510 et 610 allongés généralement cylindriques, centrés sur l'axe 1 et, dont les extrémités respectives 511, 512 et 611, 612 sont globalement arrondies..

Plus précisément, ces ballons 500 et 600 sont, respectivement, fixés sur deux tubes internes creux 520 et 620 s'étendant parallèlement à l'axe 1 et traversant axialement lesdits ballons 500 et 600. Ils comprennent, respectivement, au moins une lumière de guidage 522,622 recevant un élément de guidage de type guide 700 métallique souple d'angioplastie.

Les ballons 500 et 600 se prolongent, respectivement, à leurs extrémités proximales 511 et 611 par deux tubes de gonflage respectifs 521 et 621 s'étendant sensiblement selon l'axe 1 et adaptés pour gonfler les ballons.

De préférence, ces tubes de gonflage 521 et 621 entourent le guide 700. Plus précisément, de préférence, chacun d'entre eux comprend deux lumières: une première lumière 524, 624 qui reçoit le guide métallique 700 et débouche dans la lumière de guidage du tube interne associé 520 et 620 et une seconde lumière 525, 625 utilisée pour le gonflage qui débouche dans le volume interne des ballons 500 et 600.

Il est à noter que les tubes internes 520 et 620 et premières lumières 524 et 624 précitées sont isolés du volume interne des ballons 500 et 600.

D'autre part, les premier et second ballons 500 et 600, alignés le long de l'axe 1, sont séparés par le tube de gonflage 521 du premier ballon 500, sur une longueur de quelques mm.

De plus; ce tube de gonflage 521 se prolonge et débouche dans le tube interne 620 associé au second ballon 600, lui même se prolongeant dans le tube de gonflage 621 de ce dernier.

Ainsi, le tube de gonflage 621 du ballon 600 le plus proximal est composé, plus précisément, de trois lumières: une lumière de guidage 624 commune aux deux ballons 500 et 600, qui reçoit le guide métallique 700, une première lumière de gonflage 525 du premier ballon 500 qui débouche dans le tube interne 520 associé et une seconde lumière 625 utilisée pour le gonflage du second ballon 600 qui débouche dans le volume interne de ce ballon 600.

Par conséquent, les deux tubes de gonflage 521 et 621 associés aux deux ballons 500 et 600 sont fixés ensemble à l'extrémité proximale 611 du second ballon 600 sur une grande partie de leur longueur.

De préférence, ils sont soudés ensemble.

Ils forment alors un élément commun 410 possédant trois lumières respectives 430, 440, 450 en liaison respective avec les tubes et plus précisément les lumières de guidage 624 et de gonflage 525, 625 précitées de ceux ci.

Ainsi, les deux ballons 500 et 600 sont solidaires, joints sur la plus grande partie de leurs tubes de gonflage 521 et 621 et ils présentent une lumière de guidage 624 commune recevant le guide 700 métallique dirigeant l'ensemble dans la seconde branche B2 de la bifurcation.

Ils vont, par conséquent, se déplacer ensemble au cours d'une procédure éventuelle de pose de l'endoprothèse secondaire 200.

Par ailleurs, l'élément commun 410 du second instrument de pose 400 est muni à son extrémité proximale, opposée au second ballon 600, de deux systèmes de connexion 445 et 455 avec des sources de fluide et en liaison respective avec les lumières de gonflage 440 et 450 permettant l'expansion des ballons 500 et 600.

Il présente également des moyens de fixation réversibles et, plus particulièrement, un système de connexion 435 avec un torqueur T et en liaison avec la lumière de guidage 430 permettant la fixation du guide 700.

Ces systèmes de connexion 430, 440 et 450 peuvent être, par exemple, du type connu sous la dénomination «luer lock ».

En variante, les moyens de connexion 445 et 455 pour le gonflage des ballons précités peuvent être adaptés pour recevoir un embout de seringue de gonflage classique.

Il est important que les moyens de connexion précités 445 et 455 autorisent une expansion séparée des deux ballons 500 et 600.

Dans sa partie proximale proche des systèmes de connexion, l'élément commun 410 présente un repère de couleur R1 qui va être utilisé pour placer en bonne position l'endoprothèse secondaire 600 dans la seconde branche B2 de la bifurcation.

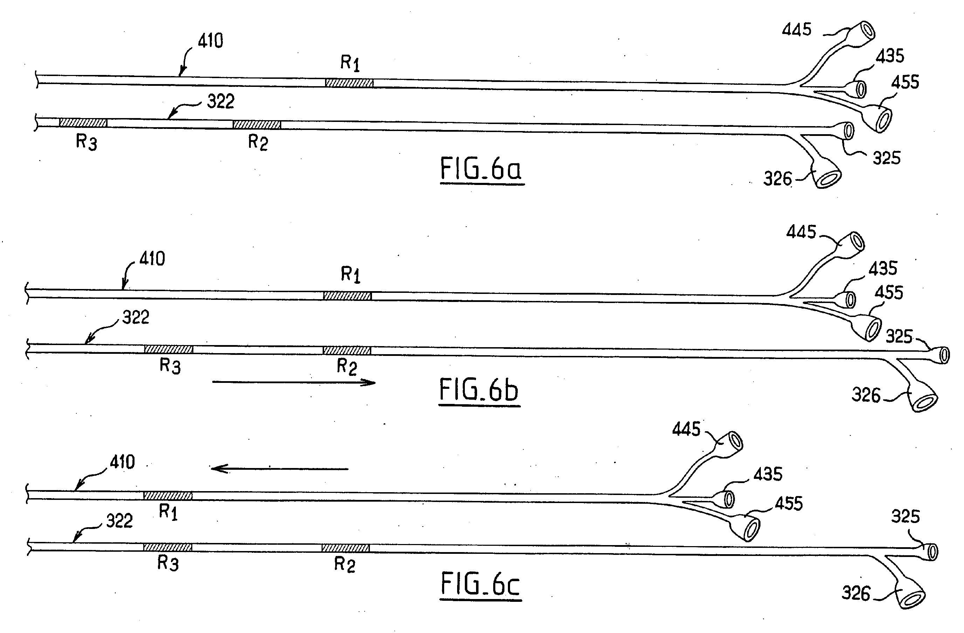

Ce repère R1, d'une longueur de l'ordre de 20 mm, est placé de façon à correspondre, lors des mouvements du second instrument de pose 400, avec les repères situés sur le premier instrument de pose 300 comme cela sera décrit plus loin en relation avec les figures 6.

De plus, chacun des deux ballons 500 et 600 est muni, de préférence, de repères radio opaque. Plus précisément, le premier ballon 500 présente un repère 540 porté par son tube interne 520 et placé à mi longueur de ce dernier tandis que le second ballon 600 présente un repère 640 situé à l'extrémité proximale 611 du ballon 600 et porté par le tube interne 620 associé.

Par ailleurs, avantageusement, le tube interne 520 du premier ballon 500 se prolonge hors de celui-ci, à son extrémité distale 512, opposée au tube de gonflage 521, par un tube appelé nez 523 du ballon 500 s'étendant le long de l'axe 1.

La lumière de guidage 522 associée au tube interne 520 débouche dans le nez 523 du premier ballon 500 et le guide 700 émerge du nez 523 par son extrémité distale 526 libre et ouverte.

Ce nez 523 du premier ballon 500 présente, également, à cette extrémité 526, des moyens de réception apte à recevoir le premier élément de guidage 800 guidant le premier instrument de pose 300.

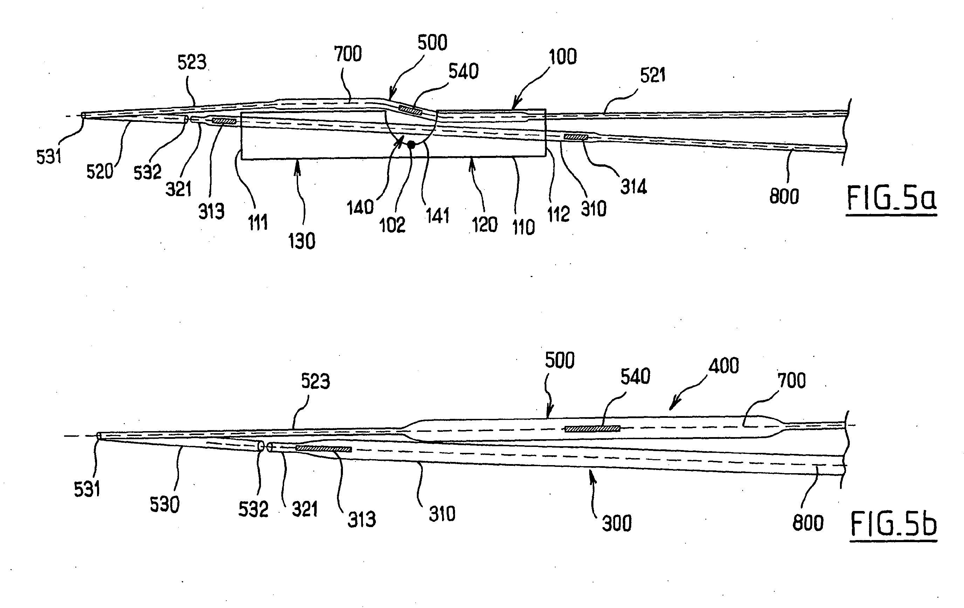

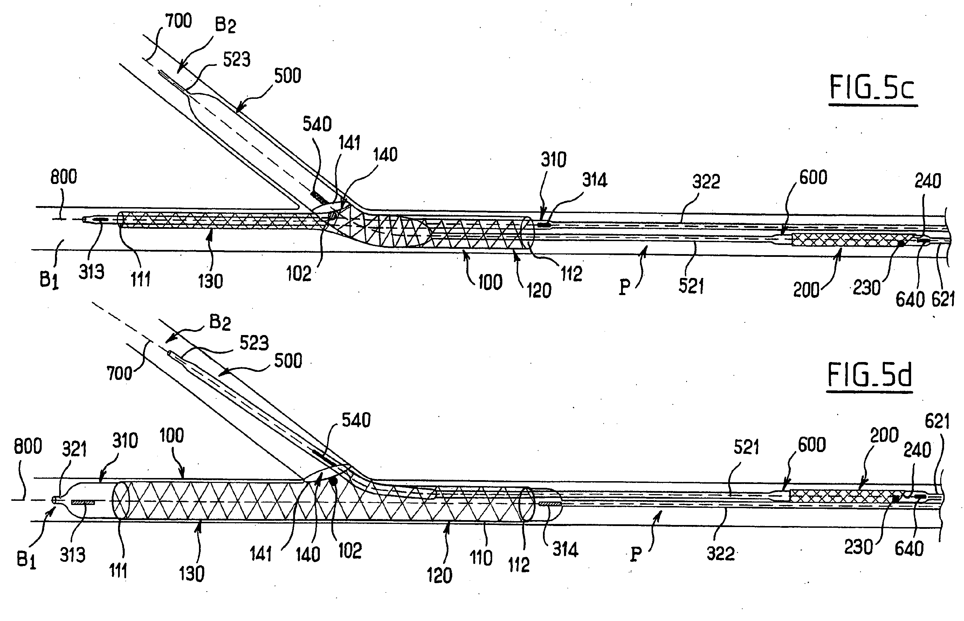

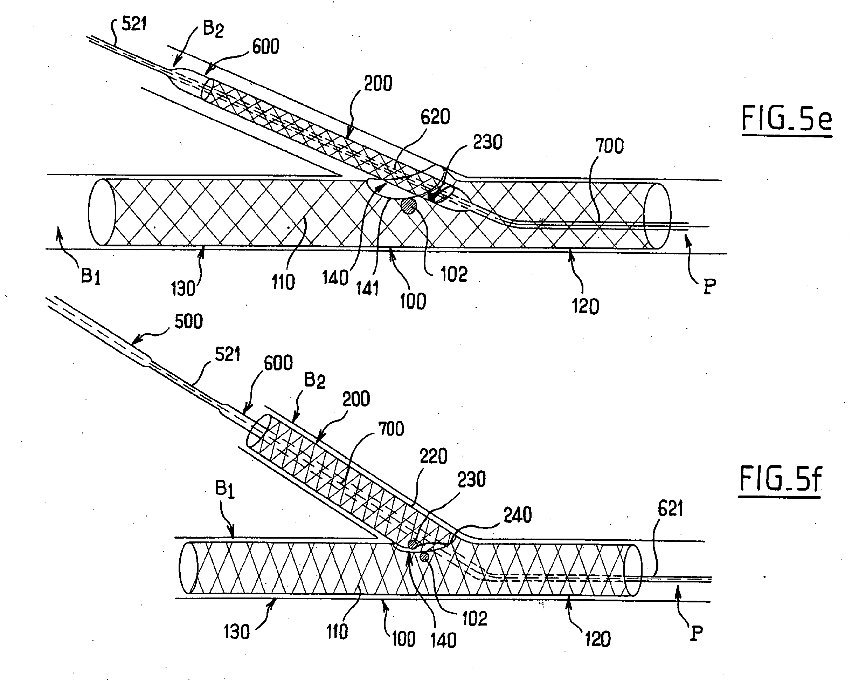

Les deux instruments de pose 300 et 400 des endoprothèses 100 et 200 vont alors pouvoir coopérer ensemble lors du placement respectif de ces dernières dans les branches B1 et B2 de la bifurcation comme cela sera décrit plus loin en relation avec les figures 5.

Ces moyens de réception comprennent un tube dit auxiliaire 530 creux, centré sur un axe 5.

Il s'étend le long de l'axe 5 de l'extrémité distale 526 du nez 523 du premier ballon 500 vers le premier ballon 500 dans une direction légèrement inclinée par rapport à l'axe 1.

Il présente une extrémité distale 531 en biseau, le biseau étant défini par un plan parallèle à l'axe 1 et perpendiculaire au plan de la feuille.

A partir de cette extrémité distale 531, il est fixé, par collage ou soudage, à l'extrémité libre 523 du nez du premier ballon 500 sur la longueur du biseau. Cette longueur est de l'ordre de quelques mm.

D'autre part, ce tube auxiliaire 530 est adapté pour recevoir, à son extrémité proximale 532, ouverte, une extrémité du mandrin 800 ou du guide métallique guidant le premier instrument de pose 300.

Concernant ce premier instrument de pose 300, illustré sur la figure 1, il comprend un ballon principal 310 centré sur un axe 2 associé à un tube interne 320, un tube de gonflage 322 et un nez 321 sensiblement identiques à ceux décrits en relation avec la figure 2 pour le premier ballon 500.

Ainsi, le premier instrument de pose 300 comprend une lumière de guidage 323 commune aux différents tubes 320 et 322 et au nez 321 apte à recevoir le mandrin 800 guidant l'instrument de pose 300 lors de sa progression dans l'artère coronaire vers la première branché B1 de la bifurcation.

Ce mandrin 800 débouche hors de l'instrument de pose 300, à l'extrémité libre 327 du nez 321 du ballon 310 et vient se placer dans le tube auxiliaire 530 du second instrument de pose 400.

Pour permettre la fixation du mandrin 800 dans le tube auxiliaire 530, le tube de gonflage 322 du ballon principal 310 est muni, à son extrémité proximale, de moyens de fixation réversibles et plus précisément d'un système de connexion 325 avec un torqueur T2 et en liaison avec la lumière de guidage 323.

Les instruments de pose respectifs 300 et 400 des deux endoprothèses principale 100 et secondaire 200 sont alors solidaires, fixés ensemble au niveau de leur nez 321 et 523 respectif.

Par ailleurs, le premier instrument de pose 300 comprend, également, un système de connexion 326 avec des sources de fluide de type luer-lock et en liaison avec une lumière de gonflage 324 permettant l'expansion du ballon principal 310.

De plus, il comporte, sur son tube de gonflage 322 deux repères de couleurs R3 et R2 séparés de quelques dizaines de mm servant de repères lors du placement de l'endoprothèse secondaire 200 dans la seconde branche B2. Le repère R3, le plus distal, et le repère R2 s'étendent, chacun, sur une longueur de l'ordre de 20 mm et sont séparés d'une distance, par exemple, de 27 mm.

Le ballon principal 310 présente, aussi, deux repères radio opaques 313 et 314 portés par son tube interne 320 et disposés, respectivement, à chacune de ses deux extrémités 312 et 311. Ces repères 313 et 314 s'étendent sur une longueur de l'ordre de 2 mm.

Selon un mode de réalisation particulier, bien entendu non limitatif, les deux instruments de pose 300 et 400 respectifs des endoprothèses principale 100 et secondaire 200 répondent aux dimensions suivantes :
- Les premier et second ballons 500 et 600 possèdent une longueur adaptée pour prendre position dans la seconde branche B2 de la bifurcation du vaisseau V tandis que ballon principal 310 possède une longueur adaptée pour prendre position dans la première branche B1 et le tronçon principal P du vaisseau V;
- Les premier et second ballons 500 et 600 ont une longueur respective de 14 mm et de 12 mm et possèdent un diamètre à l'état gonflé de l'ordre de 2.0 mm à 3.0 mm ;
- Le ballon principal 310 possède une longueur de l'ordre de 20 mm et un diamètre externe à l'état gonflé de l'ordre de 2.5 à 4 mm ;
- Le calibre externe des instruments de pose 400 et 300 solidaires à l'état affaissé des ballons 500 et 600 est, de préférence, adapté pour progresser dans un cathéter guide 6F ;
- Les lumières de guidage 624,524 et 323 des deux instruments de pose 400 et 300 ont une longueur de l'ordre de 110 à 135 cm et un diamètre interne adapté pour recevoir un guide souple ou mandrin métallique de diamètre 0.014 inches;
- Le premier ballon 500 est séparé du second 600 par son tube de gonflage 521 sur une distance de 30 à 34 mm;
- Le premier ballon 500 a un nez d'une longueur de 10.5 à 11 mm tandis que le nez 321 du ballon principal 310 s'étend sur une longueur de l'ordre de 3 mm;
- Le tube auxiliaire 530 est fixé au nez 522 du premier ballon 500 sur une longueur de l'ordre de 6 mm.

### b. Les endoprothèses

On va maintenant décrire les deux endoprothèses destinées à être placées au niveau de la zone de bifurcation comme on le voit en particulier sur la figure 4.

Concernant l'endoprothèse secondaire 200, illustrée sur les figures 3b et 4, et destinée éventuellement à être disposée dans la seconde branche B2 de la bifurcation du vaisseau V à traiter, elle comprend un tronçon 220 tubulaire centré sur un axe 4.

De préférence, le tronçon 220 est formé d'un élément tubulaire 221 ajouré en grillage de sorte que la structure du tronçon 220 autorise une expansion en section droite de celui ci.

Le dessin en maillage du tronçon peut faire l'objet de diverses variantes. Il peut s'agir par exemple d'un dessin en forme de losange ou d'hexagone, comme représenté respectivement sur les figures 3b et 4 donnant un aspect de grillage lors de leur expansion par le second ballon 600 interne, sur la paroi interne de la branche B2 de la bifurcation.

La structure de base de tels éléments tubulaires 221 expansibles et le matériau constituant ceux ci étant connus de l'homme de l'art, ces dispositions ne seront pas décrites dans le détail par la suite.

Comme illustré sur la figure 3b, l'extrémité proximale 202 du tronçon 220 destinée à être placée à l'origine de la seconde branche B2 de la bifurcation, au niveau de la carène de la bifurcation, est munie sur sa zone périphérique d'un biseau 240.

Ce biseau 240 peut être défini par un plan incliné par rapport à l'axe 4 et perpendiculaire au plan de la feuille. Ainsi, l'ouverture angulaire de la paroi du tronçon croît à partir de l'extrémité proximale 202 jusqu'à couvrir 360° c'est-à-dire une forme tubulaire complète autour de l'axe 4 à l'extrémité opposée 201.

Selon un mode de réalisation particulier, donné à titre d'exemple non limitatif, la longueur du biseau 240 est de l'ordre de 7 à 9 mm et le diamètre interne de l'endoprothèse secondaire 200 de l'ordre de 2.0 à 3.0 mm.

Une variante de réalisation prévoit une endoprothèse secondaire 200 comprenant plusieurs biseaux à son extrémité proximale 202.

D'autre part, à cette extrémité 202, l'endoprothèse 200 présente un repère 230 radio opaque. Celui ci est placé à l'extrémité 241 du biseau 240, extrémité la plus proche de l'extrémité distale 201 du tronçon 220 et destinée à être placée près de la carène de la bifurcation.

En position développée, comme illustré sur la figure 4 lorsque l'endoprothèse 200 est mise en place dans la seconde branche B2, l'extrémité proximale 202 biseautée vient se mettre en place de façon harmonieuse au niveau de l'orifice 140 de l'endoprothèse principale 100 situé à l'entrée de la seconde branche B2, afin de recouvrir au maximum la zone de bifurcation.

A propos de l'endoprothèse principale 100, illustrée sur les figures 3a et 4, elle comprend un tronçon 110 tubulaire s'étendant suivant un axe 3 et centré sur ce dernier.

Ce tronçon 110 est formé de deux portions distale 130 et proximale 120, coaxiales, destinées à être engagées, respectivement, dans la première branche B1 et dans le tronçon principal P du vaisseau V.

A une extrémité longitudinale 113 parallèle à l'axe 3, en amont de la portion distale, le tronçon 110 présente l'orifice 140 dessiné de telle sorte qu'il réalise la forme de l'entrée de la seconde branche B2 au niveau de laquelle il va venir se placer.

Il se présente, par exemple, sous la forme d'un demi arc de cercle 141 débouchant vers l'extérieur du tronçon 110.

Avantageusement, il est formé d'une maille particulière.

Cette maille est plus large que la maille définissant l'élément tubulaire 101 ajouré en grillage formant le tronçon 110.

Un tel élément 101 ayant déjà été décrit en référence avec la figure 3b pour l'endoprothèse secondaire 200, il ne sera pas décrit en détail par la suite.

En position développée, lorsque l'endoprothèse principale 100 est mise en place au niveau de la bifurcation du vaisseau V, l'orifice 140 vient se placer en regard de la seconde branche B2, prêt à recevoir le second instrument de pose 400.

Plus précisément, la maille particulière de l'orifice 140 est dessinée de telle sorte qu'elle puisse recevoir le premier ballon 500 du second instrument 400 et s'ouvrir largement sans se déformer lors de l'expansion de ce premier ballon 500 à l'entrée de la seconde branche B2 de la bifurcation.

D'autre part, elle est dessinée de manière à s'adapter à la forme de l'endoprothèse secondaire 200 sertie sur le second ballon 600 de l'instrument de pose 400, la dite endoprothèse 200 devant s'engager dans ladite maille ouverte devant l'entrée de la seconde branche B2 de la bifurcation et la traverser avant d'être disposée dans la branche B2, comme cela sera décrit en relation avec les figures 5.

Par ailleurs, un repère 102 radio opaque, d'une longueur d'environ 1 mm, est fixé sur l'orifice 140. Il peut être placé sur la périphérie de ce dernier. Par exemple,sur la figure 3a, il est situé au milieu de l'arc de cercle 141.

De façon générale, ce repère 102 radio opaque est placé de façon à correspondre lors de l'installation des deux endoprothèses 100 et 200 dans les deux branches de la bifurcation avec le repère 230 radio opaque disposé sur le biseau 240 de l'endoprothèse secondaire 200.

Dans un exemple non limitatif de l'invention, le tronçon 110 de l'endoprothèse principale 100 présente une longueur de l'ordre de 17 à 18 mm et l'orifice 140 présente un diamètre interne de l'ordre de 3 mm.

De préférence, l'endoprothèse principale 100 est une endoprothèse de type chrome cobalt.

### 2. Procédé de mise en oeuvre du système de pose d'endoprothèses pour le traitement de lésions sur des bifurcations de vaisseaux

On va maintenant décrire un procédé de mise en oeuvre du système de pose conforme à l'invention et, plus particulièrement, la mise en place d'une ou deux endoprothèses par ce système.

### a.Mise en place du système de pose des endoprothèses

En premier lieu, on place l'instrument de pose 400 de l'endoprothèse secondaire 200 dans l'endoprothèse principale 100 portée par le ballon principal 310.

Plus précisément, en référence à la figure 5a, le nez 523 du premier ballon 500 du second instrument de pose 400 est introduit à l'extrémité proximale 112 de la portion proximale 120 de l'endoprothèse principale 100 et s'engage, ensuite, hors dé l'endoprothèse principale 100, à travers la maille de l'orifice 140, jusqu'à mi longueur du tube interne 520 du premier ballon 500.

On place ensuite, par son extrémité distale, le mandrin 800 dans la lumière de guidage 323 du premier instrument de pose 300 jusqu'à ce qu'il débouche hors du nez 321 du ballon principal 310.

Comme illustré sur les figures 5a et 5b, ce mandrin 800 est alors introduit dans le tube auxiliaire 530 du second instrument dé pose 400, l'ensemble étant fixé en vissant le torqueur T2 au système de connexion 325 associé à la lumière de guidage 323.

Les deux nez 523 et 321 du ballon principal 310 et du premier ballon 500 des deux instruments de pose 300 et 400 sont ainsi maintenus solidaires l'un à l'autre.

On notera, par ailleurs, que les deux instruments de pose 300 et 400 sont, ainsi, sensiblement situés en enfilade et l'encombrement en diamètre de l'ensemble du système de pose est réduit dans l'artère coronaire.

Les deux instruments de pose 300 et 400 solidaires et les endoprothèses 100 et 200 qu'ils portent sont ensuite introduits dans le cathéter guide associé.

Le guide 700 souple, préalablement placé dans la seconde branche B2 de la bifurcation, est alors introduit par son extrémité proximale dans l'extrémité libre 526 du nez 523 du premier ballon 500 du second instrument de pose 400.

L'ensemble comprenant les deux instruments de pose 300 et 400 solidaires progresse sur ce guide 700 à travers le cathéter guide puis le tronçon principal P du vaisseau sanguin V à traiter jusqu'à atteindre la bifurcation.

Ainsi, l'ensemble tube auxiliaire 530/mandrin 800 amovible tenant solidaire les deux nez 522 et 321 du premier ballon 500 et du ballon principal 310 pendant leur progression sur le guide 700 unique vers la bifurcation permet de supprimer les risques de croisement de guides.

On arrête la progression du système de pose au niveau de la carène de la bifurcation et on libère le nez 523 du premier ballon 500 retenu par le mandrin 800 rigide.

Pour cela, dans un premier temps, on devisse le torqueur T2 du système de connexion 325 du tube de gonflage 322 du ballon principal 310, le mandrin 800 est alors libre de mouvement.

On retire, dans un deuxième temps, ce mandrin 800 du tube auxiliaire 530 libérant les deux nez 523 et 321 des deux ballons 310 et 500.

On le remplace, ensuite, dans la lumière de guidage 323 associé au ballon principal 100 par un guide souple qui est poussé dans la première branche B1 de la bifurcation.

On pousse alors sur chacun des deux guides pré positionnés dans les deux branches B1 et B2 de la bifurcation.

Les deux ballons 500 et 310, libres l'un de l'autre, et les instruments de pose associés 400 et 300 avancent sur leur guide souple respectif et se séparent respectivement, dans les deux branches B1 et B2 de la bifurcation.

Suite à ce mouvement, d'une part, la portion distale 130 de l'endoprothèse principale 100 est située dans la première branche B1, l'endoprothèse 100 ayant avancée sur son guide jusqu'à ce que l'orifice 140 soit placé à l'entrée de la seconde branche B2 de la bifurcation.

D'autre part, le nez 523 et une première moitié du premier ballon 500 traversant l'orifice 140 se sont engagés dans la seconde branche B2 de la bifurcation tandis que la seconde moitié du premier ballon 500 et le reste du second instrument de pose 400 placés dans la portion proximale 120 de l'endoprothèse principale 100 sont situés dans le tronçon principal P du vaisseau sanguin V.

Cette position précise est repérée par la correspondance des repères radio opaques 540 et 102 respectifs du premier ballon 500 et de l'orifice 140 situés au niveau de la carène de la bifurcation.

Le système de pose est maintenant en place et prêt à traiter les lésions de la bifurcation.

### b. Déploiement du système de pose des endoprothèses

Jusque là, l'ensemble des ballons est resté à l'état dégonflé.

Comme la position de l'orifice 140 et du premier ballon 500 au niveau de la carène de la bifurcation a été assurée grâce aux repères radio opaques 102 et 540, on peut procéder au gonflage des ballons.

Pour déployer le système, dans un premier temps, le premier ballon 500 du second instrument de pose 400 est gonflé par l'intermédiaire de son tube de gonflage 521.

Comme illustré sur la figure 5c, son gonflement permet d'une part, de dilater la maille de l'orifice 140 qu'il traverse et de conformer ce dernier à l'entrée de la seconde branche B2 de la bifurcation. D'autre part, il permet également de dilater la portion proximale 120 de l'endoprothèse principale 100. Ce ballon 500 est ensuite dégonflé.

Dans un second temps, comme illustré sur la figure 5d, l'inflation du ballon principal 310 permet de déployer entièrement l'endoprothèse principale 100 associée, bien ouverte.

Le ballon principal 310 est ensuite dégonflé.

A ce niveau, le grillage de l'élément tubulaire 101 de l'endoprothèse principale 100 vient recouvrir la première branche B1 de la bifurcation et le tronçon principal P du vaisseau et se place en regard de la seconde branche par son orifice 140 en présentant une maille dilatée traversée par le premier ballon 500 du second instrument de pose 400.

Dans un troisième temps, connaissant la distance séparant les deux repères de couleurs R1 et R2, respectivement portés par l'élément commun 410 du second instrument de pose 400 et par le tube de gonflage 322 du premier instrument de pose 300, on retire le ballon principal 310 de sorte que le repère de couleur R2 coïncide avec le repère de couleur R1 de l'élément commun 410, fixe comme illustré sur les figures 6a et 6b.

On réalise alors un contrôle angiographique.

Si le résultat de la pose de l'endoprothèse principale 100 est satisfaisant et optimal au niveau des deux branches B1 et B2 de la bifurcation, il n'est pas nécessaire de prévoir le déploiement de l'endoprothèse secondaire 200 dans la seconde branche B2. On retire alors tout simplement les deux instruments de pose 300 et 400 et la procédure est terminée.

Dans le cas contraire, si le résultat n'est pas optimal dans la seconde branche B2, on déplace le second instrument de pose 400 de sorte que le second ballon 600 portant l'endoprothèse secondaire 200 soit placé dans la seconde branche B2 après avoir traversé l'orifice 140 de l'endoprothèse principale 100.

Avantageusement, comme illustré sur la figure 5e, l'arrangement linéaire des deux ballons 500 et 600 du second instrument de pose 400 facilite le refranchissement de la maille de l'orifice 140 par le second ballon 600 pour implanter l'endoprothèse secondaire 200.

Connaissant la distance séparant les deux repères de couleur R3 et R2 porté par le tube de gonflage 322 du premier instrument de pose 300, on réalise ce mouvement en repoussant le second instrument de pose 400 pour faire correspondre le repère de couleur R1 avec le repère R3 comme l'illustre la figure 6c.

La position du second ballon 600 est également contrôlée par la correspondance des deux repères radio opaques 102 et 230 placés respectivement sur l'orifice 140 et sur le biseau 202 des endoprothèses principale 100 et secondaire 200 comme l'illustre les figures 5e et 5f.

Après avoir vérifié que le second ballon 600 était bien placé, on le gonfle pour déployer l'endoprothèse secondaire 200.

Celle ci se conforme, d'une part, à la paroi de la seconde branche B2 et d'autre part, vient épouser à son extrémité biseautée l'orifice 140 dilatée de l'endoprothèse principale 100.

Comme illustré sur les figures 4 et 5f, les endoprothèses principale 100 et secondaire 200 sont alors déployées pour recouvrir les deux branches B1 et B2 de la bifurcation et le tronçon principal P du vaisseau V traité.

Le traitement des lésions au niveau de la bifurcation est ainsi complet.

On réalise un dernier contrôle angiographique afin de vérifier que la disposition et le déploiement des deux endoprothèses 100 et 200 sont optimaux.

Si oui, on retire les deux instruments de pose 300 et 400 en même temps et le traitement des lésions de la bifurcation du vaisseau sanguin V grâce au système de pose conforme à l'invention est terminé.

Si non, il est aussi possible de refaire un nouveau gonflement du ballon principal 310 et du premier ballon 500, ensemble, par une technique connue dite technique de kissing-balloon qui ne sera pas décrite ici.

L'homme de l'art appréciera un système d'angioplastie pour le traitement de lésions sur des bifurcations de vaisseaux sanguins qui, par rapport aux dispositifs connus de l'état de l'art, présente un système de pose qui permet de mettre en place une endoprothèse dans une première branche B1 de la bifurcation et laisse le choix à l'opérateur d'en poser une seconde, dans la seconde branche B2, si besoin est, au cours de la même procédure.

De plus, un tel système de pose peut être utilisé de manière simple, fiable et efficace sans risque de croisement des guides métalliques dirigeant les deux endoprothèses 100 et 200 dans les branches respectives B1 et B2 de la bifurcation. En particulier, la présente invention n'est pas limitée aux dessins annexés. Les références spécifiques illustrées dans les paragraphes précédents sont des exemples non limitatifs de l'invention.

## Revendications

1. Système de traitement de lésions sur une bifurcation de vaisseau sanguin comprenant une première branche (B1) et une seconde branche (B2), le système étant **caractérisé en ce qu'**il comprend :
- une première endoprothèse (100) comprenant une portion proximale et une portion distale, ladite endoprothèse étant adaptée pour être positionnée en partie dans la première branche (B1) de la bifurcation, et comportant un orifice (140) au niveau d'une paroi latérale;
- un premier instrument de pose (300) présentant un premier axe longitudinal (2), et comprenant un ballon principal (310) adapté pour être introduit dans la première branche et pour recevoir la première endoprothèse (100) et la dilater ;
- un second instrument de pose (400) présentant un second axe longitudinal (1), comprenant :
- un premier ballon (500), ledit premier ballon étant adapté d'une part pour être introduit dans la seconde branche à travers l'orifice de la première endoprothèse (100) pour être positionné en partie dans la seconde branche (B2), et d'autre part pour dilater ledit orifice (140) et la portion proximale de la première endoprothèse, et
- un second ballon (600), aligné avec le premier ballon (500) le long du second axe longitudinal (1), adapté pour recevoir une seconde endoprothèse (200) et la dilater.

2. Système selon la revendication précédente, **caractérisé en ce que** la seconde endoprothèse (200) est adaptée pour être placée dans la seconde branche (B2) de la bifurcation et être introduite dans cette seconde branche (B2) par engagement à travers l'orifice (140) de la première endoprothèse (100) une fois celui-ci dilaté.

3. Système selon la revendication précédente, **caractérisé en ce que** l'orifice (140) possède une maille adaptée pour recevoir le premier ballon (500) et s'ouvrir largement sans se déformer lors de son expansion et apte à se conformer à la forme de la seconde endoprothèse (200) portée par le second ballon (600) qui la traverse à l'entrée de la seconde branche (B2) de la bifurcation.

4. Système selon l'une des revendications 2 ou 3, **caractérisé en ce que** la seconde endoprothèse (200) présente une extrémité proximale (202) comportant au moins un biseau (240).

5. Système selon l'une des revendications précédentes, **caractérisé en ce que** les premier et second ballons (500,600) comprennent une première lumière de guidage commune s'étendant le long dudit second axe (1), ladite première lumière de guidage étant apte à recevoir un premier élément de guidage (700) destiné à diriger l'ensemble des deux ballons (500,600) dans la seconde branche (B2) de la bifurcation.

6. Système selon la revendication précédente, **caractérisé en ce que** le premier élément de guidage (700) est dépourvu de système de connexion avec torqueur, et **en ce qu'**il est engagé dès l'origine dans l'orifice (140) de la première endoprothèse (100)

7. Système selon l'une des revendications 5 ou 6 **caractérisé en ce que** les premier et second ballons (500,600) se prolongent chacun, à une extrémité proximale (511,611), par un tube de gonflage (521,621) apte à recevoir le premier élément de guidage (700) et à gonfler le ballon (500,600), lesdits tubes de gonflage (521,621) s'étendant sensiblement parallèlement audit second axe longitudinal (1), étant fixés ensemble sur une partie de leur longueur.

8. Système selon la revendication 7 **caractérisé en ce que** les tubes de gonflage (521,621) sont soudés ensemble sur une partie de leur longueur.

9. Système selon l'une des revendications précédentes, **caractérisé en ce que** ledit ballon principal (310) est prolongé à une extrémité proximale par un tube de gonflage (322) apte à recevoir un second élément de guidage (800) et à gonfler ledit ballon (310).

10. Système selon l'une des revendications 7 à 9, **caractérisé en ce que** les tubes de gonflage (621,322) comprennent des repères de couleur.

11. Système selon les revendications 5 et 9 prises en combinaison **caractérisé en ce que** le second instrument de pose (400) possède des moyens de réception (530) aptes à recevoir en partie le second élément de guidage (800), et le premier instrument de pose (300) possède des moyens de fixation réversibles (325) dudit second élément de guidage (800) aux moyens de réception (530) de façon à maintenir solidaires les deux instruments de pose (300,400) pendant leur progression sur le premier élément de guidage (700) jusqu'à la bifurcation.

12. Système selon la revendication précédente **caractérisé en ce que** les moyens de réception (530) comprennent un tube auxiliaire creux adapté pour recevoir à une extrémité proximale (532) le second élément de guidage (800) et, pour se fixer à une extrémité distale (531) à un tube dit nez (523) comprenant la première lumière de guidage, ledit nez (523) prolongeant le premier ballon (500) à partir de son extrémité distale parallèlement au second axe (1).

13. Système selon l'une des revendications 11 ou 12, **caractérisé en ce que** les moyens de fixation réversibles (325) comprennent un système de connexion avec un torqueur (T2) et en liaison avec la seconde lumière de guidage (323) pour fixer le second élément de guidage (800) aux moyens de réception (530).

14. Système selon l'une des revendications 9 à 13, **caractérisé en ce que** le second élément de guidage (800) est un mandrin.

15. Système selon l'une des revendications 12 à 14, **caractérisé en ce que** le tube auxiliaire (530) est un tube s'étendant à partir du nez (523) dans une direction inclinée par rapport audit second axe (1) vers le premier ballon (500).

16. Système selon l'une des revendications 13 à 15, **caractérisé en ce que** le tube auxiliaire (530) comprend une extrémité distale (531) en biseau apte à être fixée par collage ou soudage au nez (523).

17. Système selon l'une des revendications précédentes, **caractérisé en ce que** chaque ballon (500,600) et chaque endoprothèse (100,200) possède au moins un repère radio opaque.

## Claims

1. A system for treating lesions on a blood vessel bifurcation, comprising a first branch (B1) and a second branch (B2), the system being **characterised in that** it comprises:
- a first endoprosthesis (100) comprising a proximal part and a distal part, said endoprostheses being adapted to be positioned in part in the first branch (B1) of the bifurcation and comprising an orifice (140) in a lateral wall;
- a first insertion instrument (300) having a first longitudinal axis (2), and comprising a main balloon (310) adapted to be introduced in the first branch and to receive the first endoprosthesis (100) and to dilate it;
- a second insertion instrument (400) having a second longitudinal axis (1), comprising:
- a first balloon (500) adapted to be introduced to the second branch (B2) via the orifice of the first endoprosthesis (100) to be positioned in part in the second branch (B2), and to dilate said orifice (140) and the proximal part of the first endoprostheses, and;
- a second balloon (600), aligned with the first balloon (500) along the second longitudinal axis (1), adapted to receive a second endoprosthesis (200) and dilate it.

2. The system as claimed in the preceding claim, **characterised in that** the second endoprosthesis (200) is adapted to be placed into the second branch (B2) of the bifurcation and to be introduced in this second branch (B2) by engagement via the orifice (140) of the first endoprosthesis (100) once said orifice is dilated.

3. The system as claimed in the preceding claim, **characterised in that** the orifice (140) has a mesh adapted to receive the first balloon (500) and open widely without deforming during its expansion and capable of conforming to the shape of the second endoprosthesis (200) carried by the second balloon (600) which passes through it at the entrance of the second branch (B2) of the bifurcation.

4. The system as claimed in any one of Claims 2 or 3, **characterised in that** the second endoprosthesis (200) has a proximal end (202) comprising at least one bevel (240).

5. The system as claimed in any one of the preceding claims, **characterised in that** the first and the second balloons (500, 600) comprise a first common guiding channel extending along said second axis (1), said first guiding channel being capable of receiving a first guiding element (700) for directing the assembly of the two balloons (500, 600) in the second branch (B2) of the bifurcation.

6. The system as claimed in the preceding claim, **characterised in that** the first guiding element (700) is deprived of a connection with torquer and **in that** it is engaged from the beginning in the orifice (140) of the first endoprosthesis (100).

7. The system as claimed in any one of Claims 5 or 6, **characterised in that** the first and the second balloons (500, 600) are each extended, at a proximal end (511, 611), by an inflation tube (521, 621) capable of receiving the first guiding element (700) and inflating the balloon (500, 600), said inflation tubes (521, 621) extending substantially parallel to said second longitudinal axis (1), being fixed together over part of their length.

8. The system as claimed in Claim 7, **characterised in that** the inflation tubes (521, 621) are welded together over part of their length.

9. The system as claimed in any one of the preceding claims, **characterised in that** said main balloon (310) is extended at a proximal end by an inflation tube (322) capable of receiving a second guide element (800) and inflating said balloon (310).

10. The system as claimed in Claims 7 to 9, **characterised in that** the inflation tubes (621, 322) comprise colour markers.

11. The system as claimed in Claims 5 and 9, **characterised in that** the second insertion instrument (400) has receiving means (530) capable of receiving in part the second guiding element (800), and the first insertion instrument (300) has reversible fixing means (325) of said second guide element (800) to the receiving means (530) so as to hold the two insertion instruments (300, 400) together during their progression on the first guiding element (700) up to the bifurcation.

12. The system as claimed in the preceding claim, **characterised in that** the receiving means (530) comprise a hollow auxiliary tube adapted to receive at a proximal end (532) the second guiding element (800) and, to be fixed at a distal end (531) to a so-called nose-tube (523) comprising the first guiding channel, said nose-tube (523) prolonging the first balloon (500) from its distal end parallel to the second axis (1).

13. The system as claimed in any one of Claims 11 or 12, **characterised in that** the reversible fixing means (325) comprise a connection system with torquer (T2) in association with the second guiding channel (323) to fix the second guiding element (800) to the receiving means (530).

14. The system as claimed in any one of Claims 9 to 13, **characterised in that** the second guiding element (800) is a mandrel.

15. The system as claimed in any one of Claims 12 to 14, **characterised in that** the auxiliary tube (530) is a tube extending from the nose-tube (523) in a direction inclined relative to said first axis (1) towards the first balloon (500).

16. The system as claimed in any one of Claims 13 to 15, **characterised in that** the auxiliary tube (530) comprises a bevelled distal end (531) capable of being fixed by adhesion or welding to the nose-tube (523).

17. The system as claimed in any one of the preceding claims, **characterised in that** each balloon (500, 600) and each endoprosthesis (100, 200) has at least one opaque radio marker.

## Patentansprüche

1. System zur Behandlung von Läsionen an einer Blutgefäßgabelung, die einen ersten Zweig (B1) und einen zweiten Zweig (B2) umfasst, wobei das System **dadurch gekennzeichnet ist, dass** es umfasst:
- eine erste Endoprothese (100), die einen proximalen Abschnitt und einen distalen Abschnitt umfasst, wobei die Endoprothese ausgelegt ist, um teilweise im ersten Zweig (B1) der Gabelung positioniert zu werden, und eine Öffnung (140) auf Höhe einer Seitenwand umfasst;
- ein erstes Verlegungsinstrument (300), das eine erste Längsachse (2) aufweist und einen Hauptballon (310) umfasst, der ausgelegt ist, um in den ersten Zweig eingeführt zu werden und um die erste Endoprothese (100) aufzunehmen und sie auszuweiten;
- ein zweites Verlegungsinstrument (400), das eine zweite Längsachse (1) aufweist, umfassend:
- einen ersten Ballon (500), wobei der erste Ballon ausgelegt ist, um einerseits in den zweiten Zweig durch die Öffnung der ersten Endoprothese (100) eingeführt zu werden, um teilweise im zweiten Zweig (B2) positioniert zu werden, und andererseits, um die Öffnung (140) und den proximalen Abschnitt der ersten Endoprothese auszuweiten, und
- einen zweiten Ballon (600), der mit dem ersten Ballon (500) entlang der zweiten Längsachse (1) ausgerichtet ist, der ausgelegt ist, eine zweite Endoprothese (200) aufzunehmen und sie auszuweiten.

2. System gemäß dem vorhergehenden Anspruche, **dadurch gekennzeichnet, dass** die zweite Endoprothese (200) ausgelegt ist, um in dem zweiten Zweig (B2) der Gabelung platziert zu werden und um in diesen zweiten Zweig (B2) eingeführt zu werden durch Einführen durch die Öffnung (140) der ersten Endoprothese (100), wenn diese einmal ausgeweitet ist.

3. System gemäß dem vorhergehenden Anspruch, **dadurch gekennzeichnet, dass** die Öffnung (140) ein Glied besitzt, das ausgelegt ist, um den ersten Ballon (500) aufzunehmen und sich weitgehend zu öffnen, ohne sich bei seiner Expansion zu deformieren und ausgelegt ist, sich an die Form der zweiten Endoprothese (200) anzupassen, die vom zweiten Ballon (600) getragen wird, der sie am Eingang des zweiten Zweigs (B2) der Gabelung durchdringt.

4. System gemäß einem der Ansprüche 2 oder 3, **dadurch gekennzeichnet, dass** die zweite Endoprothese (200) ein proximales Ende (202) aufweist, das wenigstens eine Abschrägungsfläche (240) umfasst.

5. System gemäß einem der vorhergehenden Ansprüche, **dadurch gekennzeichnet, dass** der erste und zweite Ballon (500, 600) eine erste gemeinsame Führungsöffnung umfassen, die sich entlang der zweiten Achse (1) erstreckt, wobei die erste Führungsöffnung ausgelegt ist, um ein erstes Führungselement (700) aufzunehmen, das dazu bestimmt ist, die Gesamtheit der beiden Ballone (500, 600) in dem zweiten Zweig (B2) der Gabelung zu steuern.

6. System gemäß dem vorhergehenden Anspruch, **dadurch gekennzeichnet, dass** das erste Führungselement (700) ohne Verbindungssystem mit Torquer ist und dass es vom Ursprung in der Öffnung (140) der ersten Endoprothese (100) eingeführt ist.

7. System gemäß einem der Ansprüche 5 oder 6, **dadurch gekennzeichnet, dass** der erste und zweite Ballon (500,600) jeweils verlängert sind, an einem proximalen Ende (511, 611), durch ein Aufblasrohr (521, 621), das geeignet ist, das erste Führungselement (700) aufzunehmen und den Ballon (500, 600) aufzublasen, wobei die Aufblasrohre (521, 621) sich im Wesentlichen parallel zur zweiten Längsachse (1) erstrecken, wobei sie gemeinsam auf einem Teil ihrer Länge befestigt sind.

8. System gemäß Anspruch 7, **dadurch gekennzeichnet, dass** die Aufblasrohre (521, 621) auf einem Teil ihrer Länge zusammengeschweißt sind.

9. System gemäß einem der vorhergehenden Ansprüche, **dadurch gekennzeichnet, dass** der Hauptballon (310) an einem proximalen Ende verlängert ist durch ein Aufblasrohr (322), das ausgelegt ist, ein zweites Führungselement (800) aufzunehmen und den Ballon (310) aufzublasen.

10. System gemäß einem der Ansprüche 7 bis 9, **dadurch gekennzeichnet, dass** die Aufblasrohre (621, 322) Farbmarkierungen umfassen.

11. System gemäß den Ansprüchen 5 und 9, in Kombination genommen, **dadurch gekennzeichnet, dass** das zweite Verlegungsinstrument (400) Aufnahmemittel (530) umfasst, die ausgelegt sind, das zweite Führungselement (800) teilweise aufzunehmen, und das erste Verlegungselement (300) reversible Mittel (325) zur Befestigung des zweiten Führungselements (800) an den Aufnahmemitteln (530) besitzt, um die beiden Verlegungsmittel (300, 400) während ihres Fortschreitens auf dem ersten Führungselement (700) bis zur Gabelung zusammen zu halten.

12. System gemäß dem vorhergehenden Anspruch, **dadurch gekennzeichnet, dass** die Aufnahmemittel (530) ein hohles Hilfsrohr umfassen, das ausgelegt ist, um an einem proximalen Ende (532) das zweite Führungselement (800) aufzunehmen und, um an einem distalen Ende (531) an einem Rohr genannt Nase (523) befestigt zu werden, das die erste Führungsöffnung umfasst, wobei die Nase (523) den ersten Ballon (500) von seinem distalen Ende aus parallel zur zweiten Achse (1) verlängert.

13. System gemäß einem der Ansprüche 11 oder 12, **dadurch gekennzeichnet, dass** die reversiblen Befestigungsmittel (325) ein Verbindungssystem mit einem Torquer (T2) und in Verbindung mit der zweiten Führungsöffnung (323) umfassen, um das zweite Führungselement (800) an den Aufnahmemitteln (530) zu befestigen.

14. System gemäß einem der Ansprüche 9 bis 13, **dadurch gekennzeichnet, dass** das zweite Führungselement (800) ein Mandrin ist.

15. System gemäß einem der Ansprüche 12 bis 14, **dadurch gekennzeichnet, dass** das Hilfsrohr (530) ein Rohr ist, das sich von der Nase (523) aus in eine Richtung erstreckt, die bezüglich der zweiten Achse (1) in Richtung des ersten Ballons (500) geneigt ist.

16. System gemäß einem der Ansprüche 13 bis 15, **dadurch gekennzeichnet, dass** das Hilfsrohr (530) ein distales, schräges Ende (531) umfasst, das geeignet ist, durch Kleben oder Schweißen an der Nase (523) befestigt zu werden.

17. System gemäß einem der vorhergehenden Ansprüche, **dadurch gekennzeichnet, dass** jeder Ballon (500, 600) und jede Endoprothese (100, 200) wenigstens eine strahlenundurchdringliche Markierung besitzt.
